# EUROPEAN PATENT SPECIFICATION

(11) **EP 3 870 660 B1**
(45) Date of publication and mention of the grant of the patent: **03.12.2025**
(21) Application number: 19783354.4
(22) Date of filing: 14.10.2019
(51) Int. Cl.: C09D 127/16, A61L 27/34, A61N 1/05, C08F 283/12, C09D 127/18, C09D 133/06, C09D 171/12, C09D 177/00, C09D 181/06

(54) **SILICONE MATERIAL WITH MODIFIED SURFACE TO IMPROVE SLIDING AND FRICTIONAL PROPERTIES**
SILIKONWERKSTOFF MIT MODIFIZIERTER OBERFLÄCHE ZUR VERBESSERUNG DER GLEIT- UND REIBEIGENSCHAFTEN
MATÉRIAU EN SILICONE À SURFACE MODIFIÉE PERMETTANT D'AMÉLIORER LES CARACTÉRISTIQUES DE GLISSEMENT ET DE FRICTION

(30) Priority: 25.10.2018 EP 18202647
(43) Date of publication of application: 01.09.2021
(73) Proprietor: BIOTRONIK SE & Co. KG, 12359 Berlin (DE)
(72) Inventor: BOCK, Ralf, 12051 Berlin (DE); BORCK, Alexander, 15754 Heidesee (DE)
(74) Representative: Biotronik Corporate Services SE
(86) International application number: PCT/EP2019/077737
(87) International publication number: WO 2020/083688

(56) References cited:
- WO-A1-2016/083456
- WO-A1-2017/089739
- WO-A1-2018/003822
- WO-A1-2018/176138
- JP-A- 2007 293 009
- HIROTA K ET AL: "Coating of a surface with 2-methacryloyloxyethyl phosphorylcholine (MPC) co-polymer significantly reduces retention of human pathogenic microorganisms", FEMS MICROBIOLOGY LETTERS, NO LONGER PUBLISHED BY ELSEVIER, vol. 248, no. 1, 1 July 2005 (2005-07-01), pages 37 - 45, XP027872012, ISSN: 0378-1097, [retrieved on 20050701]
- COLILLA MONTSERRAT ET AL: "The Role of Zwitterionic Materials in the Fight against Proteins and Bacteria", MEDICINES, vol. 5, no. 4, 22 November 2018 (2018-11-22), pages 125, XP093106790, DOI: 10.3390/medicines5040125

## Description

Silicones or more precisely poly(organo)siloxanes represent one of the most important material classes in the field of medical engineering. Nevertheless, attempts are being made to introduce new materials, since, besides its many advantages, silicone also has disadvantages. A main disadvantage is the relatively low resistance to wearing through. Silicone is a three-dimensionally cross-linked polymer. The desired hardness is determined partially by the network density, but primarily by aggregates, such as quartz flour. These aggregates are partly responsible for the materials having disadvantageous frictional properties of this kind. Furthermore, these aggregates may escape from the matrix under mechanical load and may then act as an abrasive substance.

Known solutions provide, for example, that the silicone is coated with outer sleeves made of a thermoplastic polyurethane (TPU), or that the silicone is replaced entirely by a TPU. The outer or cover sleeves, however, must be manufactured in a separate, demanding process, partly with very thin wall thicknesses, and then must be fitted.

A disadvantage of all thermoplastic polyurethanes (TPUs) is their insufficient hydrolysis stability and biostability, in particular on account of the polyurethane connection being unstable under hydrolysis. In particular, the metal ion oxidation leads to a premature degradation of the insulation material. Furthermore, TPUs based on aromatic isocyanates demonstrate great shortcomings in respect of biocompatibility. In order to minimise the susceptibility to hydrolysis, attempts have been made to use very lipophilic TPU formulations. The water uptake, and accordingly the hydrolysis, should thus be minimised. A further disadvantage is the high sliding friction. Furthermore, soft TPUs exhibit a tacky surface behaviour. In addition, with use of a TPU main body, a coating is essential.

TPU thus has considerable disadvantages as an alternative to silicone, in particular if TPU is used as insulation material for electrodes or electrode leads.

WO 2018/176138 A1 discloses a coating composition comprising a vinyl carboxylic acid monomer and a neutral monomer, which may be an acrylate, and a device comprising a polyacrylate coating, wherein the device may be made from a material, such as silicone.

WO 2018/003822 A1 discloses a lubricity imparting agent including a copolymer containing a 2-methacrylolyloxyethyl phosphorylcholine and photoreactive functional group-containing monomer polymer, and the use thereof in articles made of plastics

Hirota et al.: ("Coating of a surface with 2-methacryloylethyl phosphorylcholine (MCP) copolymer significantly reduces retention of human pathogenic microorganisms" FEMS MICROBIOLOGY LETTER, vol. 248, no. 1, 1 July 2005) discloses the effect of MCP-coated surfaces on the formation of bacterial biofilms.

Based on this background, it is therefore an objective of the present invention to provide a suitable material, for example as an insulation material for electrodes or electrode leads of medical implants.

This objective is achieved by the subject matter of the independent claims. Advantageous embodiments are described in the dependent claims and hereinafter.

A material according to claim 1 is therefore provided. The material according to the invention comprises a silicone, wherein a polymer is arranged on the surface of the silicone, wherein the polymer is characterised by a higher wear resistance than silicone, and the polymer is attached to the surface of the silicone by non-covalent bonds.

According to the invention it is particularly envisioned that the polymer is a methacrylic acid-methyl methacrylate copolymer which comprises a residue of a zwitterionic compound, wherein the zwitterionic compound is selected from the group comprising phosphorylcholine, sulfobetaine, carboxybetaine, sulfopyridium betaine, cysteine, phosphatidyl ethanolamine or a sulfobetaine siloxane.

Particularly, it is further envisioned that the polymer is attached to the surface of the silicone by an entanglement, looping or intermolecular interlooping of the chains of the silicone and the chains of the polymer.

In the sense of the present invention an entanglement (looping, intermolecular interlooping) is understood to mean in particular a physical/geometrical connection of two or more polymer chains which is created by crossing over the polymer chains. As a result of the entanglement, one polymer chain may be separated from the other polymer chain or the other polymer chains only with difficulty by applying force, or even may not be separated at all.

In particular, the polymer is attached to the surface of the silicone exclusively by non-covalent bonds.

The term "silicone" in the context of the present invention particularly refers to polysiloxanes with the general formula [R₂SiO]ₙ, with R being a carbon bond, in particular an alkyl, such as methyl or ethyl, or an aryl, such as phenyl.

The term "wear resistance" in the context of the present invention particularly refers to the capability of a solid surface to resist mechanical loading, in particular friction.

In particular, the surface of the silicone is coated with the polymer.

This solution according to the invention provides a silicone which not only has good friction values, but also is extremely resistant to wearing through. Due to the surface coating or the attaching of the polymer to the silicone in a manner achieved by a less complex method, the production of a cover sleeve for example and also the fitting of the cover sleeve on the main body of electrode leads of implantable devices are spared. This, combined with the excellent properties of silicone, makes the material combination or the base material an improved alternative to conventional materials.

Particularly, the polymer is a copolymer formed of two or more monomers.

Particularly, one or more monomers of the polymer are selected from the group comprising, a methacrylate (MAA) and a methyl methacrylate (MMA).

In accordance with a further embodiment of the material according to the invention, the polymer comprises 2-methacryloyloxyethyl phosphorylcholine (CAS no.: 67881-98-5) as monomer.

In accordance with a further embodiment of the material according to the invention, the silicone is a methyl silicone, in particular a polydimethylsiloxane, a vinyl-methyl silicone, a phenyl-vinyl-methyl silicone, a phenyl-modified silicone, a fluoroalkyl silicone or a fluoro-vinyl-methyl silicone.

Particularly, the polymer is a copolymer of
a. a methacrylate (methacrylic acid) and a methyl methacrylate; and
b. a zwitterionic compound, selected from phosphorylcholine, sulfobetaine, carboxybetaine, sulfopyridium betaine, cysteine, or a sulfobetaine siloxane, preferably comprising methyl or ethyl groups.

In accordance with a further embodiment of the material according to the invention, it is provided that in the polymer the methacrylic acid monomer is provided in relation to the methyl methacrylate monomer in a molar ratio in the range of from 1:1 (1 part methacrylic acid monomer to 1 part methyl methacrylate monomer) to 1:3 (1 part methacrylic acid monomer to 3 parts methyl methacrylate monomer), preferably 1:2 (1 part methacrylic acid monomer to 2 parts methyl methacrylate monomer).

In accordance with a further embodiment of the material according to the invention, the polymer comprises methacrylic acid as monomer with a mole fraction in the range of from 25% to 45%, in particular approximately 33%.

The zwitterionic compound preferably comprises at least one polymerizable molecular group, such as a vinyl group or an acryl group.

According to claim 4, a medical implant is provided, wherein the medical implant comprises the material according to the invention.

In accordance with an embodiment of the medical implant according to the invention, the medical implant comprises an electrode lead.

In the sense of the invention, an electrode lead is understood to mean in particular a device which comprises at least one elongate electrically conductive element, in particular a wire, which extends over the length of the electrode lead. In particular the electrode lead comprises an electrical, in particular tubular insulation, wherein the lumen of the insulation surrounds the at least one elongate element with the exception of the two ends. Such an insulation is also referred to as a cover sleeve.

In accordance with a further embodiment of the medical implant, the electrode lead, in particular the elongate conductive element, is sheathed at least in part with the material according to the invention.

In accordance with a further embodiment of the medical implant, the medical implant comprises a current-generating or current-emitting component (generator component, battery) and/or a current-detecting component (diagnosis component) which is connected to the electrode lead.

In accordance with a further embodiment of the medical implant it is provided that the medical implant is designed as a cardiac pacemaker, a cardioverter-defibrillator, or a neurostimulator, in particular a spinal cord stimulator.

A cardiac pacemaker is in particular a device which comprises an implantable electronic pulse generator which is connected to a patient's heart by an electrode lead.

An implantable cardioverter-defibrillator (ICD) is in particular a device which comprises a stimulation part (pulse generator) and a diagnostics part (for identifying any threat of arrhythmias), wherein the stimulation/diagnostics part, usually implanted beneath the skin in the vicinity of the left pectoral muscle, is connected to the right ventricle by an electrode lead. The electrode lead is typically guided here into the right ventricle via the upper vena cava.

The spinal cord stimulator is in particular a device for treating chronic neuropathic pain, in which a pulse generator is connected by an electrode lead to the part of the nervous system that is to be treated, for example to the posterior funiculus of the spinal cord.

In accordance with a further embodiment of the medical implant it is provided that the medical implant comprises a fixing sleeve for fixing an implantable electrode lead (electrode fixing sleeve, EFS), wherein the electrode fixing sleeve comprises or consists substantially of the material according to the invention.

In the sense of the invention the electrode fixing sleeve is understood in particular to be a special fixing sleeve which is arranged fixedly on the electrode lead at a defined point and which is then secured in the human body at a suitable position, for example by being sewn in place in the region of a muscle or a vessel.

According to claim 9, a method for producing the material according to the invention is provided. The method comprises the steps:
- providing a silicone, and
- arranging a polymer on the surface of the silicone, wherein the polymer is characterised by a greater wear resistance than the silicone, and the polymer is attached to the surface of the silicone by non-covalent bonds.

According to the invention is particularly envisioned that arranging of the polymer on the surface of the silicone comprises the following steps:
- swelling the silicone in an organic solvent yielding a swollen silicone,
- contacting the swollen silicone with the polymer, wherein the polymer is present dissolved in a suitable solvent, or
- contacting the swollen silicone with one or more precursors of the polymer, wherein the precursor of the polymer is present dissolved in a suitable solvent (inert reaction partner) or one precursor of the polymer is present dissolved in another precursor of the polymer (reactive reaction partner);
- reacting or polymerising the one or more precursors to form the polymer
- drying the silicone.

It is further particularly envisioned that the polymer is a methacrylic acid-methyl methacrylate copolymer which comprises a residue of a zwitterionic compound, wherein the zwitterionic compound is selected from the group comprising phosphorylcholine, sulfobetaine, carboxybetaine, sulfopyridium betaine, cysteine, phosphatidyl ethanolamine or a sulfobetaine siloxane

The expression "arranging a polymer" in the sense of the invention means in particular the physical attaching of the polymer to the surface of the silicone by non-covalent bonds. **In** particular the surface of the silicone is coated at least in part with the polymer.

In accordance with an embodiment of the method according to the invention, arranging of the polymer on the surface of the silicone comprises the following steps:
- dissolving the polymer in a suitable solvent yielding a polymer solution;
- applying the polymer solution to the silicone, preferably by spraying or immersion; and
- drying the silicone at a temperature in the range from room temperature to approximately 300°C, preferably at temperatures from 180°C to 260°C, and more preferably at approximately 220°C.

In the sense of the invention, "swelling the silicone" means in particular the infiltration of the organic solvent into the silicone, wherein at least the uppermost layers of the silicone experience an increase in volume. The swelling of the silicone in particular causes the silicone chains of the uppermost layers to be movable, and therefore they may entangle or loop with the polymer chains of the polymer, which is advantageous.

The swelling of the silicone also allows the polymer to be formed within the movable chains of the silicone from monomers by polymerisation, whereby an entanglement or looping of the chains of the polymer and of the silicone may also be attained here, advantageously.

A key advantage of the method according to the invention is that the method may be performed in any step of a manufacturing process for producing a device or an apparatus which is intended to comprise the material according to the invention. For example, existing electrode leads with customary silicone sheathings already may be processed by the method according to the invention so as to obtain electrode leads comprising the material according to the invention.

In the sense of the invention, "drying the silicone" means in particular the removal of the organic solvent, in particular the technically possible complete removal of the organic solvent, for example by temperature or application of a vacuum.

In order to convert or polymerise the one or more precursors into the polymer, initiators may be used, for example azobis(isobutyronitrile) (AIBN) as initiator of a radical chain reaction, for example during the polymerisation of acrylate, methacrylate (MAA), and/or methyl methacrylate (MMA).

Particularly, the one or more precursors are monomers which are selected from the following group: a methacrylate (, methacrylic acid, MAA), and/or a methyl methacrylate (MMA).

Particularly, the precursors comprise
a. methacrylate and/or methacrylate methyl ester; and
b. a zwitterionic compound selected from phosphorylcholine, sulfobetaine, carboxybetaine, sulfopyridium betaine, cysteine, phosphatidyl ethanolamine, or a sulfobetaine siloxane, preferably comprising methyl or ethyl groups,
wherein the zwitterionic compound preferably comprises a polymerizable group, for example a vinyl group or an acryl group.

Advantageously, antimicrobial surfaces preferably may be produced with the above-described zwitterionic compounds as copolymer.

The precursors preferably comprise methacrylate, ethyl methacrylate and phosphorylcholine or 2-methacryloyloxyethyl phosphorylcholine.

In accordance with a further embodiment of the method according to the invention, the organic solvent is toluene, xylene, chloroform, dichloromethane, dimethyl acetamide, dimethyl formamide, dimethyl sulfoxide, cresol, ortho-dichlorobenzene, sulfolane, tetrahydrofuran, trichlorethylene, N-methyl-2-pyrrolidone, or a methacrylate or methyl methacrylate solution. A preferred solvent for swelling the silicone is N-methyl-2-pyrrolidone, in particular in combination with n-heptane.

Further features and embodiments of the present invention will be explained hereinafter with reference to the drawings, in which:

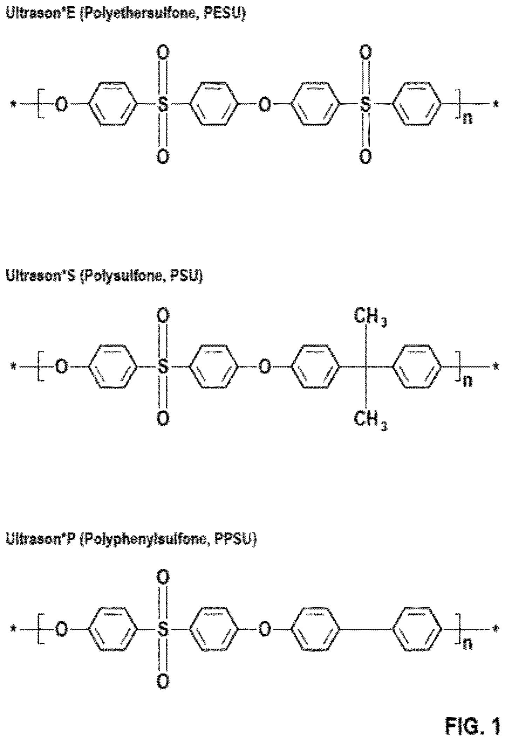

- Fig. 1: shows suitable polymers for use in the material according to the invention;
- Fig. 2: schematically shows a possible method for producing the material according to the invention;
- Fig. 3: shows an embodiment of an implant according to the invention comprising an electrode lead;
- Fig. 4: shows a further embodiment of a medical implant according to the invention comprising an electrode fixing sleeve; and
- Fig. 5: shows the results of growth efforts from cell culture attempts on different materials.

### Examples

The present invention in particular provides an insulation material which displays the known positive properties of silicone, but additionally no longer has the known disadvantages. Here, problems in particular with regard to abrasion and wearing through are reduced or avoided. Both the lead-to-lead abrasion phenomenon and inside-outside wear-through properties may thus be avoided or eliminated. On the whole, the produced materials according to the invention have a high stability with respect to frictional influences.

By way of the subject matters according to the invention, two production steps advantageously may be spared in the production of an electrode lead. The production of a correctly sized cover sleeve and the fitting of same are replaced by the method according to the invention. Customary or established electrode lead designs advantageously may be improved by the method according to the invention or adapted to market requirements. In particular, the known major problems of the implantable electrode leads, that is to say the fact that they abrade and wear through, are solved, without detriment to the handling properties.

To this end, silicone in particular is coated with suitable materials. The coating is long-lasting and is not abrasive. Apart from the sliding and frictional properties, the mechanical properties advantageously are not influenced by the coating. The surface of the silicone modified in this way makes it possible to use devices or apparatuses produced from this silicone as permanent implants.

In particular, the silicone surface may be modified such that an adhesion to tissue following implantation is reduced. An electrode lead produced from this material may also be removed from the body after a number of years, without difficulty. A further point is the antimicrobial coating. Synergies are present between the two impact directions. This means that a low, unspecific protein adsorption also constitutes an advantage in the case of infections associated with the implant.

Silicone rubber, referred to hereinafter simply as silicone, offers groups that are hardly reactive and which make possible a chemical modification. Silicone swells in some organic solvents, such as toluene or xylene. A monomer is able diffuse into this expanded matrix, which monomer is converted by radical polymerisation to form polymer chains. Following the removal of the swelling agent, these newly formed chains are then mechanically fixed in the matrix. On the other hand, suitable polymers are able to diffuse into the matrix after the swelling. Following the removal of the solvent, these polymers are then also fixed at, and on the surface.

### Determination of the swelling behaviour of silicone in different solvents

The silicone samples were weighed and kept for 24 h in the solvent to be tested. Following swelling, the sample slices were dabbed at the surface and weighed again.

The swelling factor was calculated in accordance with the following formula Q/100=(a-b)/b): Q is the swelling factor, and a is the weight after expansion, and b is the original weight. The results are shown in the following table.

**Table 1: Swelling factors of silicone in different solvents**

| DMF | Toluene | Xylene | Water | Methanol | DMSO | MMA |
|---|---|---|---|---|---|---|
| 3 | 32 | 86 | 1 | 2 | 3 | 60 |

### Test polymerisations with MMA:

i) The silicone slices were pre-swollen for 24 h at room temperature in toluene. They were then placed in the three differently concentrated polymerisation solutions. The solutions were degassed beforehand with N₂. The polymer solutions consisted of 40 ml water with either a) 1 ml MMA; b) 2.5 ml MMA, or c) 5 ml MMA. The reaction was started with 2,2'-azobis(2-methylpropionamidine)dihydrochloride at 80°C.
ii) Alternatively, the silicone samples were swollen in MMA, and then placed in the polymerisation solutions described under i).
iii) Swelling in DMF as relatively poor swelling agent over 24 h. Polymerisation was then performed in 40 ml water with 5 ml MMA. The MMA should displace the DMF because the affinity of the MMA to silicone is much higher than to the solvent water. Thereafter according to approach i).

All samples were dried at 80 °C under vacuum.

### Production of silicone rubber with MMA/MAA/PC modification:

The following exemplary embodiment describes the surface modification of a silicone rubber matrix with methyl methacrylate (MMA)/methacrylic acid (MAA) and a zwitterionic molecule.

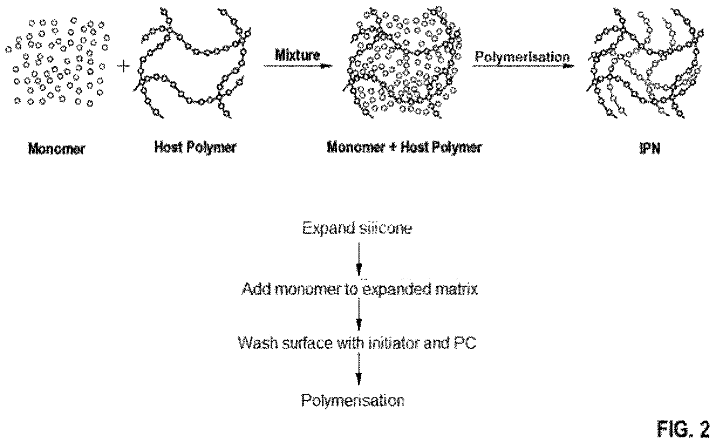

The silicone matrix was swollen using a solvent. As a result of the swelling, a volume was provided in which monomers could be converted to form prepolymers and polymers. The schema shown at the top of Figure 2 shows the build-up of this interpretation network (IP network).

A series of compounds may be used as zwitterion. A number of zwitterions will be shown in the following schema.

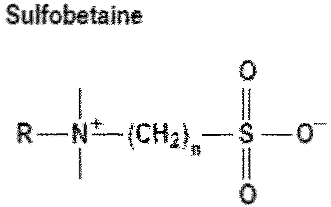

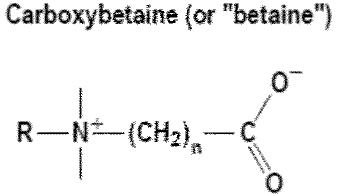

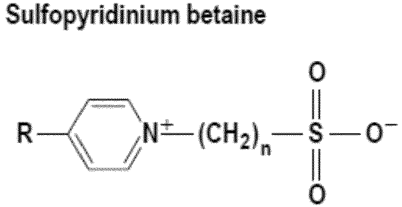

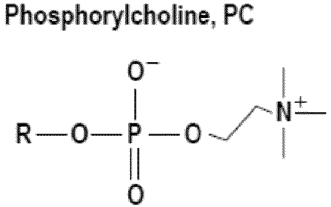

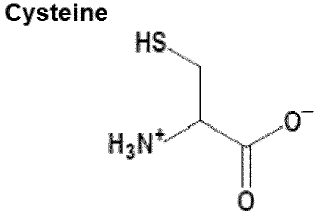

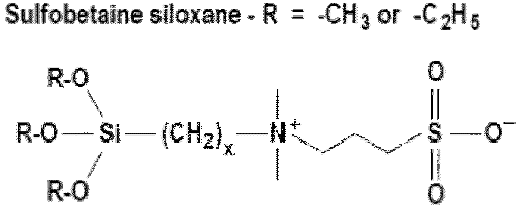

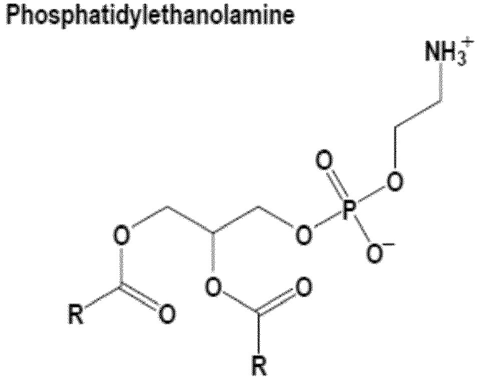

Phosphorylcholine (PC) is particularly preferred here.

In order to couple the PC to the MMA/MAA, the PC was used with a vinyl group, which was attached to the MMA/MAA prepolymer by radical polymerisation.

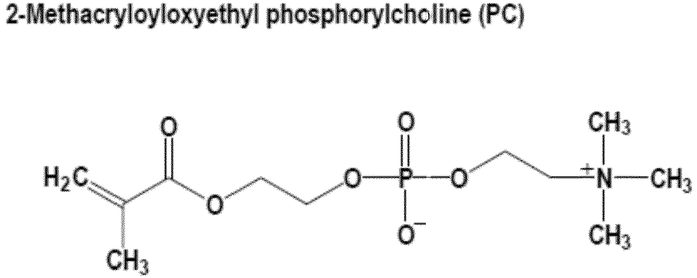

For surface modification the synthesis schema shown at the bottom in Figure 2 was applied [swelling silicone (arrow down), add monomer to swollen matrix (arrow down), wash surface with initiator and PC (arrow down) polymerisation].

### Synthesis procedure:

Substrates made of the silicone Nusil^{®} Med 45xx were added to a solution of MMA/MAA (2: 1). 100 ml MMA were mixed with 50 ml MAA and the substrates were stored for 24 h at room temperature and swollen. Following the introduction of the samples, the solution was gassed for 2 min with nitrogen so as to remove oxygen from the solution. For the further treatment, 10 mg/ml of 2,2'-azobis(2-methylpropionamidine)dihydrochloride (AIBN) were dissolved in water, and 10 mg/ml of 2-methacryoxyethyl PC were added and also dissolved at room temperature. The solution was degassed for 5 min with nitrogen. The swollen silicone samples were added to this aqueous solution and washed for 1 to 3 min. The samples were removed and incubated for 24 h at 80°C.

For repetition of the above-mentioned synthesis, an expansion solution with 40 ml MMA (methyl methacrylate, CAS: 80-62-6, Sigma Aldrich) and 20ml methacrylic acid (MAA, CAS:79-41-4, Sigma Aldrich) was produced. Silicone substrates made of Nusil MED4765 were cleaned and incubated for 3 days in the swelling solution. The silicone substrates were then removed from the expansion solution, wherein the expansion solution was then allowed to drain off. The incubated silicone substrate was then transferred into a rinsing solution, which contained 100ml demineralised water (Millipore), 100mg AIBN (radical starter) 2,2-azobis(2-methyl-propionamidine)dihydrochloride CAS: 2997-92-4 (Sigma Aldrich) and 1000mg methacryloyloxyethyl phosphorylcholine (zwitterion) CAS:67881-98-5 (Sigma Aldrich), and was rinsed for 5 min with turning. The silicone substrates were then placed on a glass plate or the like and dried for 2 days at 85°C in a furnace. The dried samples were then sterilised.

In this exemplary embodiment, one monomer is the swelling agent at the same time. Alternatively, the swelling agent may also be cyclohexane, cycloheptane, N-heptane or generally a hydrocarbon.

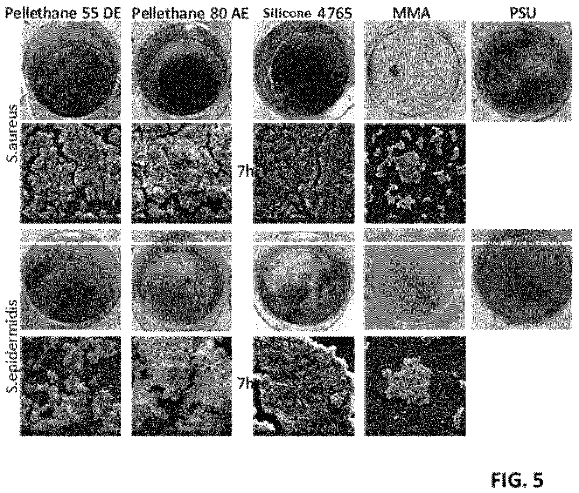

The exemplary embodiment produces surfaces which have been found to be antimicrobial (see Figure 5).

In a further exemplary embodiment, the silicone matrix was also swollen. Not only cyclohexane, cyclopentane, n-heptane or generally a hydrocarbon, but also dimethyl acetamide and N-methyl-2-pyrrolidone were used as particularly preferred solvents. In this example, it was not a monomer that was added, but a polymer, so as to produce an IP network by diffusion. PESU and PSU were used as polymers.

To this end, the silicone matrix was swollen for 48 h in the solvent at room temperature. The polymer was added as solid substance and was dissolved in the solvent at room temperature over 24 h in the presence of the silicone samples to be treated. The silicone samples were then incubated in this solution for a further 24 h and were then removed, freed from any adhering solution, and were pre-dried at 40°C for 8 h and were then freed from residual material at 40°C and 0.01 bar under vacuum.

### Exemplary embodiment of the electrode lead

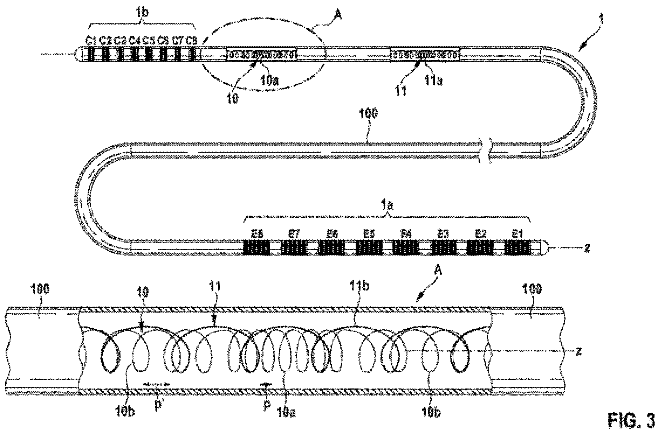

An embodiment of the implant according to the invention is shown in Figure 3 and has an electrode lead 1. The electrode lead comprises a plurality of conductors 10, 11, which extend longitudinally along the z-axis of the electrode lead 1. The conductors 10, 11 are wound here in the form of a helix around the longitudinal axis z of the electrode lead.

The electrode lead 1 has an outer (for example tubular) electrical insulation 100, which extends along the longitudinal axis z of the electrode lead 1 and surrounds the conductors 10, 11. The electrical insulation 100 here comprises the material according to the invention or consists substantially thereof.

The conductors 10, 11 are preferably formed as cables which may comprise a plurality of wires, which for example may comprise silver or tantalum or may consist substantially thereof.

In the detailed view of the region A shown in Figure 3 it is clear that the conductors 10, 11 forming a helix may be wound along the z-axis with different pitch p, p' of the helix (see reference signs 10a, 10b and 11b).

The electrode lead 1 in its distal region 1a comprises electrode terminals E1 to E8 for contacting bodily tissue. In its proximal region 1b the electrode lead also has contacts C1 to C8, by which the electrode lead may be connected to an active electrical implant, such as an implantable pulse generator - for example an implantable cardiac pacemaker or an implantable neurostimulator - or an ICD. The contacts C1 to C8 are connected here to the electrode terminals E1 to E8 by means of electrical conductors 10, 11. For this purpose the electrode lead may have more than two conductors 10, 11, as shown schematically in Figure 3. In particular, the electrode lead may have a dedicated electrical conductor for connection of the electrode pole E1 to the contact C1. The same is true also for the other electrode terminal-contact pairs E2 and C2, E3 and C3, etc.

### Exemplary embodiment of the electrode fixing sleeve

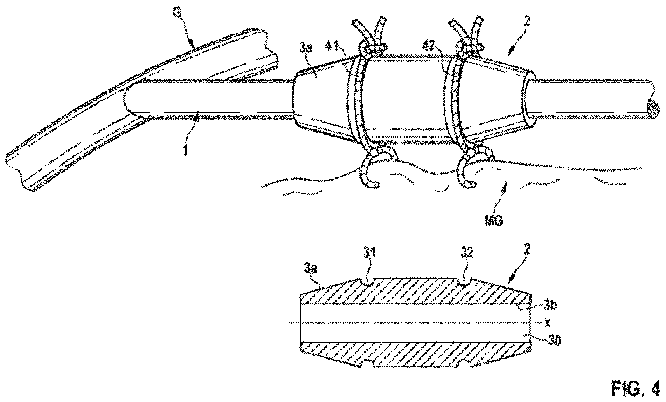

Another embodiment of the implant according to the invention relates to an electrode fixing sleeve 2, as shown by way of example in Figure 4. The fixing sleeve is used to fix an elongate element 2, here for example in the form of an implantable electrode lead 1. The electrode lead 1 has an elongate lead body, in which, in the known manner, there is arranged at least one electrical conductor - not shown here - which is connected in particular to an electrode contact, which may be arranged on a surface of the lead body so as to contact tissue of the patient. In the example according to Figure 4 the electrode lead 1 pierces a vessel G of the patient and is connected to muscle tissue MG of the patient by fixing elements 41, 42, which are secured to the fixing sleeve 2. The fixing elements 41, 42 are also used to compress or constrict the fixing sleeve 2, in such a way that the electrode lead 1 is securely clamped in a lumen 30 of the sleeve 3. The electrode lead 1 bears against an inner side 3a of the sleeve 2. The fixing elements 41, 42 are placed around the sleeve 2 in order to exert a force into corresponding grooves 31, 32, such that the sleeve may be constricted by means of the fixing elements 41, 42, resulting in a compression of the sleeve 3, at least in the region of the grooves 31, 32, such that the electrode lead 1 is securely clamped in the lumen 30 of the sleeve 2. It is provided in accordance with the invention that the electrode sleeve and/or the electrode lead comprises the material according to the invention. In particular it is provided that the electrode lead has an electrical insulation which comprises the material according to the invention, in particular on the outer surface, or the electrical insulation consists substantially of the material according to the invention. It is also provided in particular that the electrode fixing sleeve is produced substantially from the material according to the invention.

### Antimicrobial properties of the material according to the invention

Figure 5 shows the results of growth efforts with different customary materials, such as thermoplastic polyurethane elastomers (Pellethane 55DB or 80 AE), silicones (silicone 4765), and materials according to the invention (silicone modified with MMA or PSU). The materials according to the invention have a lower level of growth of *S. aureus* or *S. epidermidis.*

## Claims

1. A material comprising a silicone, **characterised in that** a polymer is arranged on the surface of the silicone, wherein the polymer is **characterised by** a higher wear resistance than the silicone, and the polymer is attached to the surface of the silicone by non-covalent bonds, wherein
the polymer is attached to the surface of the silicone by an entanglement of the chains of the silicone and the chains of the polymer, and
the polymer is a methacrylic acid-methyl methacrylate copolymer which comprises a residue of a zwitterionic compound, wherein the zwitterionic compound is selected from the group comprising phosphorylcholine, sulfobetaine, carboxybetaine, sulfopyridium betaine, cysteine, phosphatidyl ethanolamine or a sulfobetaine siloxane.

2. The material according to claim 1, **characterised in that** in the polymer the methacrylic acid monomer is present in relation to the methyl methacrylate monomer in a molar ratio in the range of from 1: 1 to 1:3, preferably 1:2.

3. The material according to any one of the preceding claims, **characterised in that** the silicone is a methyl silicone, in particular a polydimethylsiloxane, a vinyl-methyl silicone, a phenyl-vinyl-methyl silicone, a phenyl-modified silicone, a fluoroalkyl silicone, or a fluoro-vinyl-methyl silicone.

4. A medical implant comprising material according to any one of the preceding claims.

5. The medical implant according to claim 4, **characterised in that** the medical implant comprises an electrode lead.

6. The medical implant according to claim 5, **characterised in that** the electrode lead comprises an electrically insulating sheathing which comprises or consists substantially of the material according to any one of claims 1 to 3.

7. The medical implant according to claim 5 or 6, **characterised in that** the medical implant comprises a current-generating or current-emitting component and/or a current-detecting component which is connected to the electrode lead, wherein the medical implant is in particular a cardiac pacemaker, a cardioverter-defibrillator or a neurostimulator.

8. The medical implant according to any one of claims 5 to 7, **characterised in that** the implant comprises a fixing sleeve for fixing an implantable electrode lead, wherein the fixing sleeve comprises or consists substantially of a material according to any one of claims 1 to 5.

9. A method for producing a material according to any one of claims 1 to 4, comprising the steps:
- providing a silicone, and
- arranging a polymer on the surface of the silicone, including the steps of
- swelling the silicone in an organic solvent yielding a swollen silicone,
- contacting the swollen silicone with the polymer, wherein the polymer is present dissolved in a suitable solvent, or
- contacting the swollen silicone with the precursors of the polymer, wherein the one or more precursors of the polymer are present dissolved in a suitable solvent, and reacting the one or more precursors to form the polymer; and
- drying the silicone
wherein the polymer is **characterised by** a greater wear resistance than the silicone, and the polymer is attached to the surface of the silicone by non-covalent bonds, wherein the polymer is a methacrylic acid-methyl methacrylate copolymer which comprises a residue of a zwitterionic compound, wherein the zwitterionic compound is selected from the group comprising phosphorylcholine, sulfobetaine, carboxybetaine, sulfopyridium betaine, cysteine, phosphatidylethanolamine or a sulfobetaine siloxane, and wherein the precursors of the polymer comprise methacrylic acid, methyl methacrylate and a polymerisable compound with the zwitterionic compound or residue thereof.

10. The method according to claim 9, **characterised in that** the arranging of the polymer on the surface of the silicone comprises the following steps:
- dissolving the polymer in a suitable solvent yielding a polymer solution;
- applying the polymer solution to the silicone, preferably by spraying or immersion; and
- drying the silicone at a temperature in the range from room temperature to approximately 300°C, preferably at temperatures from 180°C to 260°C, and more preferably at approximately 220°C.

11. The method according to claims 9 or 10, **characterised in that** the organic solvent is toluene, cyclohexane, cycloheptane, N-heptane, Xylene or a methacrylate or methyl methacrylate solution.

## Patentansprüche

1. Material, umfassend ein Silikon, **dadurch gekennzeichnet, dass** ein Polymer auf der Oberfläche des Silikons angeordnet ist, wobei das Polymer durch eine höhere Abriebfestigkeit als das Silikon gekennzeichnet ist und das Polymer durch nichtkovalente Bindungen an der Oberfläche des Silikons angebracht ist, wobei
das Polymer durch eine Verschlaufung der Ketten des Silikons und der Ketten des Polymers an der Oberfläche des Silikons befestigt ist und
das Polymer ein Methacrylsäure-Methylmethacrylat-Copolymer ist, das einen Rest einer zwitterionischen Verbindung umfasst, wobei die zwitterionische Verbindung ausgewählt ist aus der Gruppe bestehend aus Phosphorylcholin, Sulfobetain, Carboxybetain, Sulfopyridiumbetain, Cystein, Phosphatidylethanolamin oder einem Sulfobetainsiloxan.

2. Material nach Anspruch 1, **dadurch gekennzeichnet, dass** das Polymer des Methacrylsäure-Monomers in Bezug auf das Methylmethacrylat-Monomer in einem Molverhältnis im Bereich von 1:1 bis 1:3, vorzugsweise 1:2, vorhanden ist.

3. Material nach einem der vorhergehenden Ansprüche, **dadurch gekennzeichnet, dass** das Silikon ein Methylsilikon ist, insbesondere ein Polydimethylsiloxan, ein Vinyl-Methyl-Silikon, ein Phenyl-Vinyl-Methyl-Silikon, ein Phenyl-modifiziertes Silikon, ein Fluoralkylsilikon oder ein Fluor-Vinyl-Methyl-Silikon.

4. Medizinisches Implantat, umfassend ein Material nach einem der vorhergehenden Ansprüche.

5. Medizinisches Implantat nach Anspruch 4, **dadurch gekennzeichnet, dass** das medizinische Implantat eine Elektrodenleitung umfasst.

6. Medizinisches Implantat nach Anspruch 5, **dadurch gekennzeichnet, dass** die Elektrodenleitung eine elektrisch isolierende Abschirmung umfasst, die das Material nach einem der Ansprüche 1 bis 3 umfasst oder im Wesentlichen daraus besteht.

7. Medizinisches Implantat nach Anspruch 5 oder 6, **dadurch gekennzeichnet, dass** das medizinische Implantat eine stromerzeugende oder stromemittierende Komponente und/oder eine stromdetektierende Komponente, die mit der Elektrodenleitung verbunden ist, umfasst, wobei das medizinische Implantat insbesondere ein Herzschrittmacher, ein Kardioverter-Defibrillator oder ein Neurostimulator ist.

8. Medizinisches Implantat nach einem der Ansprüche 5 bis 7, **dadurch gekennzeichnet, dass** das Implantat eine Fixierhülse zum Fixieren einer implantierbaren Elektrodenleitung umfasst, wobei die Fixierhülse ein Material nach einem der Ansprüche 1 bis 5 umfasst oder im Wesentlichen daraus besteht.

9. Verfahren zum Herstellen eines Materials nach einem der Ansprüche 1 bis 4, umfassend die Schritte:
- Bereitstellen eines Silikons und
- Anordnen eines Polymers auf der Oberfläche des Silikons, was die folgenden Schritte beinhaltet:
- Aufquellen des Silikons in einem organischen Lösungsmittel, was zu einem aufgequollenen Silikon führt,
- Inkontaktbringen des aufgequollenen Silikons mit dem Polymer, wobei das Polymer in einem geeigneten Lösungsmittel aufgelöst vorhanden ist, oder
- Inkontaktbringen des aufgequollenen Silikons mit den Vorläufern des Polymers, wobei der eine oder die mehreren Vorläufer des Polymers in einem geeigneten Lösungsmittel aufgelöst vorhanden sind, und Umsetzen des einen oder der mehreren Vorläufer, um das Polymer zu bilden; und
- Trocknen des Silikons,
wobei das Polymer durch einen größeren Verschleißwiderstand als das Silikon gekennzeichnet ist und das Polymer durch nicht kovalente Bindungen an der Oberfläche des Silikons angebracht ist, wobei das Polymer ein Methacrylsäure-Methylmethacrylat-Copolymer ist, das einen Rest einer zwitterionischen Verbindung umfasst, wobei die zwitterionische Verbindung ausgewählt ist aus der Gruppe umfassend Phosphorylcholin, Sulfobetain, Carboxybetain, Sulfopyridiumbetain, Cystein, Phosphatidylethanolamin oder einem Sulfobetainsiloxan, und wobei die Vorläufer des Polymers Methacrylsäure, Methylmethacrylat und eine polymerisierbare Verbindung mit der zwitterionischen Verbindung oder dem Rest davon umfassen.

10. Verfahren nach Anspruch 9, **dadurch gekennzeichnet, dass** das Anordnen des Polymers auf der Oberfläche des Silikons die folgenden Schritte umfasst:
- Auflösen des Polymers in einem geeigneten Lösungsmittel, was zu einer Polymerlösung führt;
- Auftragen der Polymerlösung auf das Silikon, vorzugsweise durch Sprühen oder Tauchen; und
- Trocknen des Silikons bei einer Temperatur im Bereich von Raumtemperatur bis ungefähr 300 °C, vorzugsweise bei Temperaturen von 180 °C bis 260 °C und weiter bevorzugt bei ungefähr 220 °C.

11. Verfahren nach Anspruch 9 oder 10, **dadurch gekennzeichnet, dass** das organische Lösungsmittel Toluol, Cyclohexan, Cycloheptan, N-Heptan, Xylol oder eine Methacrylat- oder Methylmethacrylat-Lösung ist.

## Revendications

1. Matériau comprenant une silicone, **caractérisé en ce qu'**un polymère est agencé sur la surface de la silicone, dans lequel le polymère est **caractérisé par** une résistance à l'usure supérieure à celle de la silicone, et le polymère est attaché à la surface de la silicone par des liaisons non covalentes, dans lequel
le polymère est attaché à la surface de la silicone par un enchevêtrement des chaînes de la silicone et des chaînes du polymère, et
le polymère est un copolymère acide méthacrylique-méthacrylate de méthyle qui comprend un résidu d'un composé zwitterionique, dans lequel le composé zwitterionique est choisi dans le groupe comprenant la phosphorylcholine, la sulfobétaïne, la carboxybétaïne, la bétaïne de sulfopyridium, la cystéine, la phosphatidyléthanolamine ou une sulfobétaïne siloxane.

2. Matériau selon la revendication 1, caractérisé à ce que dans le polymère le monomère d'acide méthacrylique est présent en relation avec le monomère de méthacrylate de méthyle dans un rapport molaire dans la plage allant de 1:1 à 1:3, de préférence 1:2.

3. Matériau selon l'une quelconque des revendications précédentes, **caractérisé en ce que** la silicone est une méthylsilicone, en particulier un polydiméthylsiloxane, une vinyl-méthylsilicone, une phényl-vinyl-méthylsilicone, une silicone modifiée par phényle, une fluoroalkylsilicone ou une fluoro-vinyl-méthylsilicone.

4. Implant médical comprenant un matériau selon l'une quelconque des revendications précédentes.

5. Implant médical selon la revendication 4, **caractérisé en ce que** l'implant médical comprend un câble d'électrode.

6. Implant médical selon la revendication 5, **caractérisé en ce que** le câble d'électrode comprend une gaine électriquement isolante qui comprend ou est essentiellement constituée du matériau selon l'une quelconque des revendications 1 à 3.

7. Implant médical selon la revendication 5 ou 6, **caractérisé en ce que** l'implant médical comprend un composant générant du courant ou émettant du courant et/ou un composant détectant le courant qui est connecté au câble d'électrode, dans lequel l'implant médical est en particulier un stimulateur cardiaque, un défibrillateur automatique ou un neurostimulateur.

8. Implant médical selon l'une quelconque des revendications 5 à 7, **caractérisé en ce que** l'implant comprend un manchon de fixation destiné à fixer un câble d'électrode implantable, dans lequel le manchon de fixation comprend ou est essentiellement constitué d'un matériau selon l'une quelconque des revendications 1 à 5.

9. Procédé de production d'un matériau selon l'une quelconque des revendications 1 à 4, comprenant les étapes consistant à :
- fournir une silicone, et
- agencer un polymère sur la surface de la silicone, incluant les étapes consistant à
- faire gonfler la silicone dans un solvant organique pour obtenir une silicone gonflée,
- mettre en contact la silicone gonflée avec le polymère, dans lequel le polymère est présent sous forme dissoute dans un solvant adapté, ou
- mettre en contact la silicone gonflée avec les précurseurs du polymère, dans lequel le ou les précurseurs du polymère sont présents sous forme dissoute dans un solvant adapté, et faire réagir le ou les précurseurs pour former le polymère ; et
- faire sécher la silicone
dans lequel le polymère est **caractérisé par** une résistance à l'usure supérieure à celle de la silicone, et le polymère est attaché à la surface de la silicone par des liaisons non covalentes, dans lequel le polymère est un copolymère acide méthacrylique-méthacrylate de méthyle qui comprend un résidu d'un composé zwitterionique, dans lequel le composé zwitterionique est choisi dans le groupe comprenant la phosphorylcholine, la sulfobétaïne, la carboxybétaïne, la bétaïne de sulfopyridium, la cystéine, la phosphatidyléthanolamine ou une sulfobétaïne siloxane, et dans lequel les précurseurs du polymère comprennent l'acide méthacrylique, le méthacrylate de méthyle et un composé polymérisable avec le composé zwitterionique ou un résidu de celui-ci.

10. Procédé selon la revendication 9, **caractérisé en ce que** l'étape consistant à agencer le polymère à la surface de la silicone comprend les étapes suivantes consistant à :
- dissoudre le polymère dans un solvant adapté pour obtenir une solution de polymère ;
- appliquer la solution de polymère à la silicone, préférentiellement par pulvérisation ou immersion ; et
- faire sécher la silicone à une température dans la plage allant de la température ambiante à environ 300 °C, de préférence à des températures allant de 180 °C à 260 °C, et plus préférentiellement à environ 220 °C.

11. Procédé selon la revendication 9 ou 10, **caractérisé en ce que** le solvant organique est le toluène, le cyclohexane, le cycloheptane, le N-heptane, le xylène ou une solution de méthacrylate ou de méthacrylate de méthyle.
